(19) Europäisches Patentamt
European Patent Office
Office européen des brevets

(11) **EP 0 530 531 B1**

(12) **EUROPÄISCHE PATENTSCHRIFT**

(45) Veröffentlichungstag und Bekanntmachung des Hinweises auf die Patenterteilung:
**18.11.1998 Patentblatt 1998/47**

(51) Int. Cl.$^6$: **A61K 7/00**, A61K 7/48

(21) Anmeldenummer: **92113441.7**

(22) Anmeldetag: **06.08.1992**

(54) **Konservierungsmittelfreie Kosmetika**

Cosmetic without preservatives

Composition cosmétique exempte d'agents conservateurs

(84) Benannte Vertragsstaaten:
**AT BE CH DE DK ES FR GB GR IE IT LI LU NL PT SE**

(30) Priorität: **14.08.1991 DE 4126969**

(43) Veröffentlichungstag der Anmeldung:
**10.03.1993 Patentblatt 1993/10**

(73) Patentinhaber: **Benckiser N.V.**
**1118 BH Schiphol Airport (NL)**

(72) Erfinder:
• **Wäschenbach, Guido, Dr.**
**W-6800 Mannheim 81 (DE)**
• **Baust, Heinrich**
**W-6831 Plankstadt (DE)**

(74) Vertreter:
**Lederer, Franz, Dr. et al**
**Lederer, Keller & Riederer**
**Patentanwälte**
**Prinzregentenstrasse 16**
**80538 München (DE)**

(56) Entgegenhaltungen:
**EP-A- 392 426        EP-A- 442 519**
**EP-A- 494 391        DE-A- 1 492 030**
**DE-A- 3 232 136      FR-A- 2 075 400**
**FR-A- 2 208 642      FR-A- 2 593 509**
**GB-A- 2 206 048      US-A- 4 606 913**

• **COSMETICS & TOILETRIES Bd. 96, Nr. 8, August 1981, Seiten 55 - 57 DISAPIO ET AL. 'new silicone emulsifier technology'**
• **PATENT ABSTRACTS OF JAPAN vol. 5, no. 164 (C-76)(836) 21. Oktober 1981**
• **PATENT ABSTRACTS OF JAPAN vol. 10, no. 172 (C-460)(2228)**
• **PATENT ABSTRACTS OF JAPAN vol. 11, no. 365 (C-460)(2812)**

**Beschreibung**

Die vorliegende Erfindung betrifft konservierungsmittelfreie, insbesondere selbstkonservierende Kosmetika in Form von Emulsionen vom Typ O/W oder W/O.

Unter Kosmetika im Sinne der vorliegenden Erfindung werden "leave-on"-Produkte verstanden, insbesondere solche Emulsionen, die als Tagescreme, Nachtcreme, Babycreme, Body Lotion, Sonnenschutz-Lotion oder -Creme, After-Sun-Lotion oder -Creme formuliert werden. Die Erfindung eignet sich insbesondere für sogenannte Naturkosmetika, die aus der Sicht der Landesuntersuchungsämter die Grundbedingung "frei von chemischen Konservierungsstoffen" erfüllen.

Wasserhaltige Emulsionen dieser Art, insbesondere wenn sie den für Körperpflegemittel üblichen pH-Bereich zwischen 5 und 8 aufweisen, sind ideale Nährböden für Mikroorganismen, die infolge ihrer hohen Stoffwechselaktivität die Produkte innerhalb kurzer Zeit verderben können. Solche Produkte müssen daher im allgemeinen Konservierungsmittel enthalten.

Konservierungsmittel sind jedoch häufig Ursachen von Verträglichkeitsproblemen (Überempfindlichkeit, Allergien) und bedingen für Verbraucher und Hersteller immer ein Restrisiko in bezug auf Produktsicherheit und Akzeptanz. Außerdem werden Konservierungsmittel häufig in den kosmetische Grundlagen abgebaut und sie können durch Reaktion mit anderen Rezepturkomponenten farbliche Probleme aufwerfen. Da für die Konservierungsmittel in den verschiedenen Ländern unterschiedliche Zulassungsregelungen bestehen, ist der Hersteller von konservierungsmittelhaltigen Produkten in seinen Export-Aktivitäten eingeschränkt.

Aus der aufgezeigten Problematik ergibt sich, daß konservierungsmittelfreie Produkte mit eigenem, ausreichendem antimikrobiellen Potential für Verbraucher und Hersteller gleichermaßen eine Problemlösung von höchstem Interesse darstellen.

Aufgabe der vorliegenden Erfindung ist es daher, Kosmetika in Form von Emulsionen vom Typ O/W oder W/O zur Verfügung zu stellen, die konservierungsmittelfrei sind und selbstkonservierende Eigenschaften besitzen.

Es ist bekannt, Magnesiumsalze, beispielsweise $MgSO_4$ x 7 $H_2O$ in O/W- bzw. W/O-Emulsionen in Mengen zwischen 0,2 und 0,7 Gew. % zur Emulsions-Stabilisierung einzusetzen. In solchen Mengen besitzen die Magnesiumsalze jedoch keine selbstkonservierenden Eigenschaften.

Ein Marktprodukt in Form einer W/O-Creme, welches konservierungsmittelfrei ist und 0,3 Gew. % $MgSO_4$ x 7 $H_2O$ enthält, ist durch externe Keimbelastung hochgradig infizierbar trotz seiner ansonsten ungünstigen Wachstumsbedingungen wie niedriger Wassergehalt und W/O-Emulsionstyp.

Es wurde nun überraschenderweise gefunden, daß die gegenständlichen Kosmetika konserviert werden können durch Einverleibung von 4-35 Gew. %, vorzugsweise 5-15 Gew. % Magnesiumchlorid (berechnet als $MgCl_2$ x 6 $H_2O$).

Die DE-B1-29 27 278 offenbart Haarwaschmittel, die anorganische Salze zur Regelung der Viskosität enthalten können.

Aus der DE-A1-38 20 330 sind Haarpflegeemulsionen bekannt, die ein anionisches Tensid in einer Menge von 0,1-6,5 Gew. % enthalten. Solche Haarpflegeemulsionen sind "rinse-off"-Produkte und keine Kosmetika im Sinne der vorliegenden Erfindung.

Die US-P 4 744 924 offenbart einen kosmetischen Waschrohstoff, der zur Einstellung besonderer rheologischer Eigenschaften Natrium- und Magnesium-Ionen enthalten soll.

Die DE-A1-23 00 594 offenbart Hautbehandlungsmittel, insbesondere Kosmetika, die als obligatorischen Bestandteil Harnstoff enthalten. Diesen Kosmetika werden ferner anorganische Salze, vorzugsweise Natriumchlorid oder Magnesiumchlorid in relativ hohen Mengen zugesetzt, weil diese in Kombination mit dem Harnstoff einen synergistischen Effekt in Bezug auf die Wasserretention ergeben. Ein konservierender Effekt eines solchen Salzzusatzes wird dagegen nicht offenbart.

"Patent Abstracts of Japan", Vol. 5, No. 164 (C-76) (836) offenbart Kosmetika, die ein anorganisches Salz enthalten, z.B. Kaliumsulfat. Magnesiumchlorid wird nicht erwähnt. Die in dieser Druckschrift offenbarten Kosmetika enthalten 0,4-9,8 Gew. % eines mehrwertigen Metallsalzes einer 10-22 C-gesättigten oder ungesättigten Fettsäure, beispielsweise Ammoniumstearat, als ein anionisches Tensid.

FR-A-25 93 509 offenbart ein nichtionisches Tensid und in einem Beispiel wird eine Creme offenbart, die Magnesiumsulfat in einer Menge von 2% enthält.

US-A-4 606 913 offenbart Emulsionen, die zur Verbesserung der Emulsionsstabilität einen Elektrolyt, ausgewählt aus der Gruppe bestehend aus Kaliumsulfat und Magnesiumsulfat, enthalten.

DE-A-32 32 136 offenbart ein Gemisch enthaltend Meersalz und/oder Salinensalz, welches zur Hand- oder Fußpflege benutzt werden kann. Es werden eine Handpflege- oder Fußpflegecreme sowie Körperpflegemittel beschrieben, die aus einem Gemisch von 65-23% einer 5-20%-igen wäßrigen NaCl-Lösung, 5-15% Aqualose und 10-30% Eusarin bestehen.

In ihrer allgemeinsten Form betrifft die vorliegende Erfindung die Verwendung von Magnesiumchlorid, in einer Menge von 4-35 Gew. %, vorzugsweise 5-15 Gew. % zur Konservierung von Kosmetika, die frei von herkömmlichen Konservierungsmitteln sind und keine anionischen Tenside enthalten.

Gegenstand der Erfindung sind somit konservierungsmittelfreie Kosmetika in Form von Emulsionen vom Typ O/W oder W/O üblicher Zusammensetzung, die 4-35 Gew. %, vorzugsweise 5-15 Gew. % Magnesiumchlorid (berechnet als $MgCl_2$ x 6 $H_2O$) jedoch keine anionischen Tenside enthalten.

Die Obergrenze muß so gewählt werden, daß ein Aussalzen vermieden wird, und liegt im allgemeinen bei 20, vorzugsweise 15 Gew. %. Wenn Magnesiumchlorid 6 aq eingesetzt wird, liegt dessen Menge bei 4-35 Gew. %, vorzugsweise 5-15 Gew. %.

In weiterer Ausbildung der Erfindung wurde gefunden, daß das erfindungsgemäß eingesetzte Magnesiumchlorid auch zur Herstellung von Desodorierungsmitteln für den Körper verwendet werden kann. Desodorierungsmittel für den Körper wirken auf die Schweiß-zersetzenden Bakterien auf der Haut ein. In Desodorierungsmitteln, die einen Gehalt an Magnesiumchlorid gemäß vorliegender Erfindung aufweisen, wirkt dieses Salz also nicht nur als Hilfsmittel zur Selbstkonservierung der Produkte, sondern als eigentlicher Wirkstoff zur Bekämpfung der Hautbakterien. Die Erfindung umfaßt also auch Desodorierungsmittel, unabhängig davon, ob sie als Emulsionen vorliegen oder nicht, die den erfindungsgemäßen Gehalt an Magnesiumchlorid aufweisen.

Die erfindungsgemäßen Kosmetika enthalten keine anionischen Tenside und sind frei von Harnstoff.

Die erfindungsgemäßen Kosmetika enthalten im allgemeinen 1-25 Gew. %, vorzugsweise 5-10 Gew. % eines geeigneten Emulgators.

Geeignete Emulgatoren, die als Monosysteme und in unterschiedlichen Kombinationen zur Herstellung von autosterilen Hautpflege-Emulsionen eingesetzt werden können, sind in der nachstehenden Tabelle A zusammengestellt.

Tabelle A

Geeignete Emulgatorsysteme- und Emulsionsstabilisatoren

Polyoxyethylen-glycerin-fettsäureester

Glycerin-mono; di- und triester

Fruchtsäureester der Mono-Diglyceride

Glykol-Fettsäure-Ester

Ethylenoxidaddukte von hydriertem Rizinusöl

Siliciumorganische Polymere

Sorbitan-Fettsäureester

Sorbitan-Fettsäureester-ethoxylate

Fettalkohole

Wachse

Lanolin

Lanolinalkohole

Fettalkohol-Ethoxylate

Polyoxyethylen-Fettsäure-Ester

Polyoxyethylen Sorbitester

Polyoxyethylen Sorbitol

Alkylpolyglukoside

Phosphorsäureester

Organische Polymere

Acrylamid Copolymere, z.B. Carbomer 934, 940, 941

Hydrokolloide

Silica

Bentonite

Ca- und Mg-Seifen

Besonders bevorzugte Emulgatorsysteme sind für

<u>O/W-Systeme:</u>

Mischungen aus Glycerinmono- und Distearaten und Stearylalkohol und Stearylalkohol-Ethoxylaten (7EO und 10EO).
und für

<u>W/O-Systeme:</u>

Emulgator-Compounds aus der Gruppe der Polysiloxanpolyether-copolymere unter Zusatz von ethoxylierten Glycerinestern und Fettsäureestern, z.B. die Kombination aus Cetyl Dimethicone Copolyol, Polyglyceryl-4-Isostearat und Hexyl Laurat.
Die erfindungsgemäßen Kosmetika können auch 1-35 Gew. %, vorzugsweise 5-20 Gew. % eines Lipids enthalten. Für die Herstellung der autosterilen Hautpflege-Emulsionen geeignete Lipide sind in der nachstehenden Tabelle B zusammengestellt.

Tabelle B

Geeignete Lipide

| FETTSÄUREESTER |
| --- |
| Triglyceride, Monester gesättigter bzw. ungesättigter, linearer bzw. verzweigter $C_8/C_{24}$-Fettsäuren mit linearen bzw. verzweigten $C_3/C_{18}$-Alkoholen |
| FETTALKOHOLE |
| Oleylalkohol, Octyldodecanol |
| KOHLENWASSERSTOFFE |
| Paraffinöle, Vaseline, wachsartige Iso- und N-Paraffine |
| SILICIUMVERBINDUNGEN |
| Siliciumorganische Öle und Wachse, Dimethylsiloxane |
| STERINE |
| Lanolin, Lanolinalkohole, Derivate |

Dem Verwendungszweck entsprechend können den erfindungsgemäßen Kosmetika unterschiedliche Wirkstoffe einverleibt werden. Einige typische Wirkstoffe sind in der nachstehenden Tabelle C aufgeführt.

Tabelle C

WIRKSTOFFE

- Vitamine

- Kräuterextrake

- Organextrakte

- Proteine

- kühlende Substanzen, z.B. Menthol, Alkohol etc.

- durchblutungsfördernde Substanzen

- Antiphlogistika

- ätherische Öle

- UV-Filter

- Farbpigmente

# EP 0 530 531 B1

Tabelle C (fortgesetzt)

## WIRKSTOFFE

- Selbstbräunungsmittel

- Moisturizer, NMF-Substanzen

- Phospholipide

- Sphingolipide

Eine erfindungsgemäß bevorzugte Hautpflege-Emulsion vom O/W-Typ enthält 5-10 Gew. %, vorzugsweise 7-9 Gew. % einer Emulgatorkombination aus Glycerinmono-Distearat, Stearylalkohol und Stearylalkoholethoxylat (7 mol EO und 10 mol EO) und 5-20 Gew. % Magnesiumchlorid 6 aq.

Eine erfindungsgemäß bevorzugte Hauptpflege-Emulsion vom W/O-Typ enthält 5-10 Gew. %, vorzugsweise 5-7 Gew. % einer Emulgatorkombination aus Cetyl Dimethicone Copolyol, Polyglyceryl-4-Isostearat und Hexylaurat sowie 5-20 Gew. % Magnesiumchlorid 6 aq.

Erfindungsgemäß bevorzugte O/W- und W/O-Emulsionen zur Hautpflege bestehen aus 10-40 % Ölphase (Emulgatoren, Lipide, Öl-lösliche Additive) und 90-60 % Wasserphase (Wasser, wasserlösliche Additive).

Bei emulgatorfreien Schüttelmixturen kann die Ölphase 5-80 % und die Wasserphase 95-20 % betragen.

Im allgemeinen enthalten die erfindungsgemäßen Emulsionen 65-82 Gew. % Wasser.

In der nachstehenden Tabelle D werden erfindungsgemäße Modellrezepturen (B, C, E und F) verglichen mit Vergleichsrezepturen, die nur ganz wenig Magnesiumsulfat als Stabilisator enthalten (A und D) sowie mit einem konservierungsmittelfreien Marktprodukt (G).

EP 0 530 531 B1

## Tabelle D   Autosterile Hautpflege-Emulsionen
### (Modellrezepturen)

Rezepturbeispiele A - G

| Emulsionstyp | O/W-Emulsionen | | | W/O-Emulsionen | | | Vergleich: W/O-Emulsion |
|---|---|---|---|---|---|---|---|
| Rohstoffe | **A** E % | **B** E % | **C** E % | **D** E % | **E** E % | **F** E % | **G** * |
| Emulgatorkombination aus: Glycerinmono-distearat, Stearylalkohol und Stearylalkoholethoxylat (7EO und 10EO) | 8,00 | 8,00 | 8,00 | 0,00 | 0,00 | 0,00 | Markenprodukt W/O-Creme |
| Isopropylmyristat | 6,00 | 6,00 | 6,00 | 0,00 | 0,00 | 0,00 | *Ohne Konser- |
| Ethylhexylstearat | 6,00 | 6,00 | 6,00 | 0,00 | 0,00 | 0,00 | vierungsstoffe" |
| Emulgatorkombination aus: Cetyl Dimethicone Copolyol und Polyglyceryl-4 Isostearate und Hexyl Laurate | 0,00 | 0,00 | 0,00 | 5,00 | 5,00 | 5,00 | CTFA-Deklarierung: Water, Paraffin-Oil, Vaseline, Glycerin, |
| Decamethylcyclopentasiloxan | 0,00 | 0,00 | 0,00 | 10,00 | 10,00 | 10,00 | Lanolin Alcohol (Eucerit) |
| Octamethylcyclopentasiloxan | 0,00 | 0,00 | 0,00 | 10,00 | 10,00 | 10,00 | Ceresin, Paraffin Wax, |
| Magnesiums... 'at x 7 H2O | 0,50 | 0,00 | 0,00 | 0,50 | 0,00 | 0,00 | Octyldodecanol, |
| Magnesiumchlorid x 6 H2O | 0,00 | 5,00 | 10,00 | 0,00 | 10,00 | 20,00 | Decyl Oleate. Mg/Al-Stearates, |
| Parfümöl | 0,10 | 0,10 | 0,10 | 0,10 | 0,10 | 0,10 | Magnesium Sulfate. Citric Acid, Panthenol, |
| Rezeptur-Wasser | ad 100% | ad 100% | ad 100% | ad 100% | ad 100% | ad 100% | Fragrance |
| **Wassergehalt Emulsion** | **80%** | **78%** | **75%** | **75%** | **70%** | **66%** | **67%** |

*endprodukt*

* G = Mark[t] für Konservierungsmittel-freie W/O-Cremes

Analyse ergab: Wassergehalt:          67,0%

              Summe Mg-Gehalt bezogen auf MgSO4x7H2O:          0,3%

## Tabelle D (Fortsetzung)

### Mikrobiologische Ergebnisse
(Modellrezepturen A - G)

| | O/W-Emulsionen | | | W/O-Emulsionen | | | Markenprodukt (W/O-Creme) |
|---|---|---|---|---|---|---|---|
| | A | B | C | D | E | F | G |
| Mg-Salz-Gehalt | 0,50% MgSO4 x 7aq | 5% MgCl2 x 6aq | 10% MgCl2 x 6aq | 0,50% MgSo4 x 7aq | 10% MgCl2 x 6 aq | 20% MgCl2 x 6 aq | ca. 0,3% MgSO4 x 7H2O |
| Mischsuspension Bakterien<br>Staph. aureus<br>Escherichi coli<br>Pseudomonas aeruginosa — Keimreduktion der belasteten Emulsionen nach 14 Tagen auf: | $2,0 \times 10^4$ KBE/ml | $5,6 \times 10^2$ KBE/ml | $1,6 \times 10^3$ KBE/ml | ca. 0,5 Mio KBE/ml | $1,6 \times 10^2$ KBE/ml | $5,0 \times 10^1$ KBE/ml | ca. 0,5 Mio KBE/ml |

Belastung der Emulsionen mit $2,5 \times 10^7$ Bakterien/ml

Die Erfindung wird durch die nachstehenden Rezepturbeispiele 1-6 näher erläutert.

| Beispiel 1: Tagescreme | |
|---|---|
| Emulgatorgrundlage aus: | % |
| Stearylalkohol, Stearylalkohol 7 EO, Stearylalkohol 10 EO | 8,0 |
| Isopropylstearat | 5,0 |
| Cetyl-stearylalkohol-Isononoat | 5,0 |
| Hexyl-Laurat | 4,5 |
| Stearylalkohol | 2,0 |
| 2-Pyrrolidon-5-carbonsäure, Na-Salz | 0,5 |
| Glycerin | 1,5 |
| $MgCl_2$ x 6 aq | 7,0 |
| Wasser | 66,2 |
| Parfümöl | 0,3 |
| | 100,0 |

| Beispiel 2: Nachtcreme | |
|---|---|
| Emulgatorgrundlage aus: | % |
| Paraffinöl, Paraffinwachs, Ozokerit, Glycerin-Oleat und Lanolin Alkohol, | 20,0 |
| Sorbitansesquioleat | 1,0 |
| Ethylhexylstearat | 3,0 |
| Mandelcel | 3,0 |
| Ceramid | 0,3 |
| Lanolin 50 | 1,0 |
| Mikrokristallines Salbenwachs | 2,0 |
| Glycerin | 1,5 |
| $MgCl_2$ x 6 aq | 6,0 |
| Wasser | 62,0 |
| Parfümöl | 0,2 |
| | 100,0 |

| Beispiel 3: Baby-Creme | |
|---|---|
| | % |
| Mono-, Di- und Tri-(alkyltetraglykolether)-o-phosphorsäure-ester | 1,0 |

(fortgesetzt)

| Beispiel 3: Baby-Creme | |
|---|---|
| | % |
| Cetylstearylalkohol, 12 EO | 4,5 |
| Lanolin flüssig | 2,5 |
| Paraffinöl PL | 12,5 |
| Paraffinwachs | 3,5 |
| Carbomer 941 | 1,2 |
| 1,2-Propylenglykol | 1,0 |
| Tris(hydroxymethyl)aminomethan | 1,4 |
| $MgCl_2$ x 6 aq | 5,0 |
| Allantoin | 0,1 |
| Kamillenextrakt | 1,0 |
| Wasser | 66,0 |
| Parfümöl | 0,3 |
| | 100,0 |

| Beispiel 4: Body Lotion | |
|---|---|
| | % |
| Polyoxyethylen-glyzerin-monostearat | 2,6 |
| Glycerinmono-distearat | 2,4 |
| Sojalecithin, hydriert | 1,0 |
| Paraffinöl PL | 18,0 |
| Carbomer 941 | 1,0 |
| 1,2-Propylenglykol | 1,0 |
| $MgCl_2$ x 6 aq | 5,0 |
| Wasser | 67,7 |
| Triethanolamin 99%ig | 1,0 |
| Parfümöl | 0,3 |
| | 100,0 |

| Beispiel 5: Sonnenschutzlotion | |
|---|---|
| Emulgatorgrundlage aus: | % |
| Cetyl Dimethicone Copolyol, Polyglyceryl-4-Isostearate und Hexyl-Laurat | 6,0 |
| Decamethylcyclopentasiloxan | 6,0 |

# EP 0 530 531 B1

(fortgesetzt)

| Beispiel 5: Sonnenschutzlotion | |
|---|---|
| Emulgatorgrundlage aus: | % |
| Capryl/Caprinsäure-triglycerid | 3,0 |
| Paraffinöl | 3,0 |
| Octyl-methoxycinnamat | 3,0 |
| Hydroxymethoxybenzophenon | 1,0 |
| $MgCl_2$ x 6 aq | 5,0 |
| Wasser | 72,7 |
| Parfümöl | 0,3 |
| | 100,0 |

| Beispiel 6: After Sun-Emulsion | |
|---|---|
| | % |
| POE Fettsäure-Ester | 6,0 |
| Cetylalkohol | 2,0 |
| Stearinsäure | 2,0 |
| Paraffinöl per liquidum | 6,0 |
| Sorbit 70%ig | 3,0 |
| Allantoin | 0,3 |
| $MgCl_2$ x 6 aq | 8,0 |
| Wasser | 72,4 |
| Menthol | 0,1 |
| Parfümöl | 0,2 |
| | 100,0 |

**Patentansprüche**

1. Konservierungsmittelfreie Kosmetika in Form von Emulsionen vom Typ O/W oder W/O üblicher Zusammensetzung, dadurch gekennzeichnet, daß sie 4-35 Gew.-%, vorzugsweise 5-15 Gew.-% Magnesiumchlorid (berechnet als $MgCl_2$ x 6 $H_2O$) jedoch keine anionischen Tenside enthalten.

2. Kosmetika nach Anspruch 1 in Form einer Tagescreme, Nachtcreme, Babycreme, Body Lotion, Sonnenschutz-Lotion oder -Creme, After-Sun-Lotion oder -Creme.

3. Kosmetika nach einem der vorgehenden Ansprüche, dadurch gekennzeichnet, daß sie 1-25 Gew.-%, vorzugsweise 5-10 Gew.-% eines geeigneten Emulgators enthalten.

4. Kosmetika nach einem der vorgehenden Ansprüche, dadurch gekennzeichnet, daß sie 1-35 Gew.-%, vorzugsweise 5-20 Gew.-% eines Lipids enthalten.

5. Kosmetika nach einem der vorgehenden Ansprüche in Form einer Hautpflege-Emulsion vom O/W-Typ, enthaltend 5-10 Gew.-%, vorzugsweise 7-9 Gew.-% einer Emulgatorkombination aus Glycerinmono-Distearat, Stearylalkohol und Stearylalkoholethoxylat (7 mol EO und 10 mol EO) und 5-20 Gew.-% Magnesiumchlorid 6 aq.

6. Kosmetika nach einem der Ansprüche 1-4 in Form einer Hautpflege-Emulsion vom W/O-Typ, enthaltend 5-10 Gew.-%, vorzugsweise 5-7 Gew.-% einer Emulgatorkombination aus Cetyl Dimethicone Copolyol, Polyglyceryl-4-Isostearat und Hexylaurat sowie 5-20 Gew.-% Magnesiumchlorid 6 aq.

## Claims

1. Preservative-free cosmetics in the form of emulsions of the oil-in-water or water-in-oil type of customary composition, characterized in that they contain 4-35 wt. %, preferably 5-15 wt. %, of magnesium chloride (calculated as $MgCl_2 \cdot 6H_2O$), however do not contain anionic surfactants.

2. The cosmetics as claimed in claim 1 in the form of a day cream, night cream, baby cream, body lotion, sun-protection lotion or cream, after-sun lotion or cream.

3. The cosmetics as claimed in any of the preceding claims, characterized in that they contain 1-25 wt. %, preferably 5-10 wt. %, of a suitable emulsifier.

4. The cosmetics as claimed in any of the preceding claims, characterized in that they contain 1-35 wt. %, preferably 5-20 wt. %, of a lipid.

5. The cosmetics as claimed in any of the preceding claims in the form of a skin-care emulsion of the oil-in-water type, containing 5-10 wt. %, preferably 7-9 wt. %, of an emulsifier combination of glycerin mono-distearate, stearyl alcohol and stearyl alcohol ethoxylate (7 mols EO and 10 mols EO) and 5-20 wt. % of magnesium chloride $\cdot$ $6H_2O$.

6. The cosmetics as claimed in any of claims 1-4 in the form of a skin-care emulsion of the water-in-oil type, containing 5-10 wt. %, preferably 5-7 wt. %, of an emulsifier combination of cetyl dimechicone copolyol, polyglyceryl-4-isostearate and hexyl laurate, and 5-20 wt. % of magnesium chloride $\cdot$ $6H_2O$.

## Revendications

1. Produits cosmétiques dépourvus d'agent conservateur, sous la forme d'émulsions du type huile/eau ou eau/huile de composition usuelle, caractérisés en ce qu'ils contiennent de 4 à 35% en poids, de préférence, de 5 à 15% en poids, de chlorure de magnésium (calculés sous forme de $MgCl_2$ x 6 $H_2O$), mais cependant pas de tensioactifs anioniques.

2. Cosmétiques suivant la revendication 1 sous la forme d'une crème de jour, d'une crème de nuit, d'une crème pour bébés, d'une lotion corporelle, d'une lotion de protection contre le soleil ou d'une crème de protection contre le soleil, d'une lotion ou d'une crème après exposition au soleil.

3. Cosmétiques suivant l'une quelconque des revendications précédentes, caractérisés en ce qu'ils contiennent de 1 à 25% en poids, de préférence, de 5 à 10% en poids, d'un émulsif approprié.

4. Cosmétiques suivant l'une quelconque des revendications précédentes, caractérisés en ce qu'ils contiennent de 1 à 35% en poids, de préférence, de 5 à 20% en poids, d'un lipide.

5. Cosmétiques suivant l'une quelconque des revendications précédentes, sous la forme d'une émulsion pour soins de la peau du type huile/eau, contenant 5 à 10% en poids, de préférence, 7 à 9% en poids, d'une combinaison d'émulsifs constituée de mono-distéarate de glycérine, d'alcool stéarylique et d'éthoxylate d'alcool stéarylique (7 moles d'OE et 10 moles d'OE) et 5 à 20% en poids de chlorure de magnésium 6 aq.

6. Cosmétiques suivant l'une quelconque des revendications 1 à 4, sous la forme d'une émulsion pour soins de la peau du type eau/huile, contenant de 5 à 10% en poids, de préférence, de 5 à 7% en poids, d'une combinaison d'émulsifs de cétyldiméthiconecopolyol, de poly(4-isostéarate de glycéryle) et de laurate d'hexyle, ainsi que de 5 à 20% en poids de chlorure de magnésium 6 aq.